# EUROPEAN PATENT SPECIFICATION

(11) **EP 3 454 805 B1**
(45) Date of publication and mention of the grant of the patent: **26.08.2020**
(21) Application number: 17727813.2
(22) Date of filing: 12.05.2017
(51) Int. Cl.: A61F 13/00, A61K 47/02, A61F 7/00, A61K 9/70, A61N 1/00

(54) **DEVICE FOR DELIVERING MEDICAMENTS OR ACTIVE INGREDIENTS**
VORRICHTUNG ZUR ABGABE VON MEDIKAMENTEN ODER VON WIRKSTOFFEN
DISPOSITIF D'ADMINISTRATION DE MÉDICAMENTS OU DE PRINCIPES ACTIFS

(30) Priority: 12.05.2016 GB 201608315
(43) Date of publication of application: 20.03.2019
(73) Proprietor: Grafmed, Altrincham, Cheshire WA15 6RP (GB)
(72) Inventor: LEIGH, Martin, Altrincham Cheshire WA15 6RP (GB)
(74) Representative: Wilson Gunn
(86) International application number: PCT/EP2017/061539
(87) International publication number: WO 2017/194781

(56) References cited:
- WO-A1-2013/140176
- CN-A- 1 383 800
- CN-U- 203 564 412
- CN-U- 204 682 701

## Description

The present invention relates to a device for the delivery of one or more medicaments or other active ingredients to a human or animal body, and which also enables the controlled sequential delivery of a plurality of different medicaments in a predetermined sequence through thermal or electrical management.

### BACKGROUND OF THE INVENTION

Many different devices for the delivery of medicaments or active ingredients to a human or animal body are known. However, the medical industry is always on the lookout for new and improved devices for the delivery of the same.

There are few single devices which enable a patient to administer one or more medicaments or active ingredients to themselves, as well as enable the controlled sequential administration of a number of them. The development of a new such device, especially one which is easy for a non-medical practitioner to control, would be highly sought after.

Background prior art is disclosed in WO2013/140176 A1. Interesting prior art is also revealed in CN 204682701 U.

Therefore, in accordance with the invention, there is provided a device for delivering one or more medicaments or active ingredients to a human or animal body according to the subject-matter of claim 1.

The metal composite material may be any suitable conductive composite material which contains one or more metals selected from copper, zinc, platinum, silver, gold, nickel, gallium, or cobalt, or combinations of any two or more thereof.

Typically, the metal composite material comprises an amount of copper, more typically at least about 40 or 50% by weight of the first layer.

According to one embodiment, the first layer comprises at least about 40 or 50% of one particular 'primary' metal by weight of the first layer, and further comprises one or more other metals overlying the primary metal which act to encase the primary metal to prevent oxidization. Typically, the metal which constitutes at least about 40 or 50% by weight of the first layer is copper.

The graphene in the first layer may be present in the form of an outer layer on top of the overall metal composite material, added by any suitable method, including but not limited to, spraying; or it may be integrated into the metal composite material, acting as a layer on top of the metal which constitutes at least about 40 or 50% by weight of the first layer, to provide effective conductivity and a sterile environment.

The first layer is typically in the form of a mesh structure.

The graphene composite may be any suitable composite material containing graphene, such as graphene oxide. Typically, the graphene and/or graphene composite are contained within a fine mesh structure.

As used herein, the terms active ingredients' and 'medicaments' are interchangeable. However, they are also intended to include active ingredients which are suitable for administration to the human or animal body which may not necessarily be administered for purely therapeutic purposes.

The one or more active ingredients, or medicaments, are able to be released from the device, one at a time in a predetermined sequence, upon application to the device of thermal change, through body heat, light, or one or more electric charges of varying strengths. These electric charges may be derived from, or created by, the device itself, or alternatively derived from an external source of power such as laser lighting, or alternatively using body heat from a host to activate the device layers. The device may also include the ability to use the same system of delivery using body heat or laser or LED light by the effect of mesh flexing within the layered device where there may not be a connected source of power.

The one or more medicaments are each contained within the second layer in individual sections thereof, each being separately suspended in place within a graphene composite web structure, which can resemble a spider's web-type arrangement, for example. The graphene structure may have any shape or geometry, but may be, by way of non-limiting example, hexagonal, such as a series of concentric hexagons.

The medication suspended in the web would be initially added to the web by means of chemical or electronic inducement through submersive baths of the graphene, allowing the medication to electronically bind to the device in the reverse process of discharge. The higher the inducement, the more capacity the structure will have to be able to absorb medication. This would work in the reverse when administrated. The suspended medication would be added in the form of minute 'blocks' which will be lifted and attracted to the graphene structure by electronic or chemical inducement with the medication being attracted to localised or general areas of the device. The device can map specific areas of a wound and use this mapped area as a guide for medical application. The structure would be stored in a sealed environment until used.

The medicament is administrated by an induced low electrical current released at varying voltage levels, by a powerful lighting source or, induced by the patient's or host's own body heat. Upon a changed state of body heat (*e.g.* a burns wound where natural heat is released through the body) or low-level electric charge or current to the device, the physical structure of the graphene in the second layer changes, condensing and structurally condensing or expanding the lower structural layers of the graphene, opening up a pathway for a first medicament stored in the device to be released.

When the device contains more than one medicament, the medicaments can be released sequentially upon application of electrical charges of increased strength. Early release of medication can be induced as an option by body heat, or external light; after this level of release, further low voltage supply release will then travel through limited highly conductive parts of the device web and change the device layering structure, typically the outer sections, releasing only a part of the stored medication, such as the first medicament. A slightly increased electrical charge is carried further through the medical structure, facilitating the release of a second medicament; and a further increase in the electrical charge travels still deeper into the structure, facilitating the release of a third and subsequent medicaments. This allows for a staged and sequential release of each of the stored medicaments.

However, if desired, and if sufficient charge can be generated initially, then two or three different medicaments could be released or administered simultaneously.

The increased electrical charge also results in an increased degree of tightening of the device on the patient, increasing the pressure on a body part. It is also envisaged that a sufficiently increased level of tightening may also allow for nano injection of medicaments into a patient from the device, as there is a higher degree of contact between the device and the patient's skin.

The tightening is achieved by the flexing of the mesh material of the first layer tessellating or flexing and changing physical structure. This interaction of the layers as they are subject to heat and charge changes their physical state by flexing and expanding in a vertical or horizontal contouring to the patient /host skin and body part shape. This distortion is achieved by the use of the conductive metal composite material, including graphene that is able to operate under very low levels of current. These will typically be in the form of a mesh or lattice structure and will be optimized for the specific 'load' of the medication or change of state that is required for medication delivery.

Depending on the supplied source of power, light, heat, electrical, the device structure may provide the tensile strength of a plaster cast, with its sterile and waterproof qualities making it suitable for bespoke production in all environments.

After treatment has been completed, the device may be physically released from a patient by reverse unzipping the fastening and enabling the current generation to flow in the opposite direction causing the device to slacken and reduce in height or width, thus rendering the device inert, whereupon it returns to its resting malleable state for removal or re-application.

The graphene structure has several areas that are accessed only by strength of current flow. Therefore, a smaller charge will not have the conductivity current to reach the furthest point of release within the structure. Like a web only the stronger voltages will reach the furthest parts of the grid. These sequential structures may be suspended and stacked either vertically from the point of application or in regular geometrical patterns on one level next to one and other or a combination of both. The final structure of the medical layer will be determined by the method of attachment and strength of dose required.

Before application, the medication is induced into the structure the reverse way it is medicated, electronically and or chemically, The structure will either be 'dipped' or chemically induced to allow the structure to collect the medication this can be varied in strength by the choice of composite or the density of graphene based material used to receive and 'load' the medication into the structure. Third party composites may be used to enable this process depending upon the medication content.

The device has its own power supply, using advanced battery cell technology such as hemp, graphene optimized coin cells or graphene supercapacitors for rapid power discharge and the subsequent controlled release of charge is regulated by a localized control on the fastener device the process is initiated by a coin cell which provides the battery mechanism its initial power to start the manual process of fastening to create the charge. In order to secure the device around a body part of a person, the device is equipped with one or more fasteners. Typically, the one or more fasteners are in the form of a zip (or zipper), or, they are in the form of a rigid or flexible structure where they consist of two electrodes divided by the zip head membrane "separator", an electrolyte that allows ions to glide back and forth between the electrodes. In the zip fastener, the interlocking teeth are typically alternating anodes and cathodes.

When a battery is charging, lithium ions are released from the positive electrode, or cathode, which consists of a lithium alloy, commonly lithium cobalt oxide or lithium iron phosphate and enhanced by graphene oxide. Other identified metal oxides which can be used for enhancement include MnO₂, Co₃O₄, SnO₂, and ZnO₂. They are drawn toward the negatively charged electrode, called the anode, which is usually made of graphite. There they snuggle in between the graphite's sheets of carbon atoms. Voltage from the coin battery drives the initial ionic mass migration into the material. The anode and cathode interact with the fastener membrane to provide a charge as well as a binding join between the device's edges, which can act as a zip head, or zip pull, or fasten over. The zip is able to act as a primary power source for the device as it contains cell coin battery to initiate the battery, it can be supplemented by external power if required. Integrated directly into the zip's teeth, the conductive material of the medical device enables current to travel directly into mesh layer which carries it into forming the contouring shape of the device. From this point the operator has the option of a second phase of power indicated on the fastener as to the level of current to be release into the release of medication in a controlled way, either through a secondary pulse of power or DC low current depending upon the medical delivery requirement.

The zip mechanism may be enhanced by the use of graphene enhanced supercapacitors for rapid charging and release of electrical charge.

When the zip head is moved up and down the teeth, the faster the charge is generated, and the more charge is generated. These power levels are able to be represented on the zip head, to enable controlled discharge to induce medication levels. The localized voltage range will operate between 0.5V to a maximum of 12V. Smaller sections of material may be 'patchworked' to concentrate power into particular areas, allowing for supplied electrical charge to be very specific and targeted with delivery of the medication.

The 'zip head' will contain up to any number of power level indicators providing the administrator the knowledge of what they can administrate in terms of power and therefore medication. As the 'zip fastener' is moved these power levels will indicate the available discharge charge and using a simple secondary release button for each or all of the stages (should the mechanism be creating power above the desired voltage) will further indicate the level of power and therefore medication available to the operator.

Typically, the device can be used to store and dispense up to about six different medicaments, more typically from one to about four different medicaments. Devices containing two and three medicaments are also envisaged.

In the embodiments of the invention where one or two, or even three, medicaments are stored in the device, then these medicaments may be able to be released using only the electrical charge generated by the device itself. For the embodiments where more than three medicaments are stored in the device (and also some embodiments where three medicaments are stored), then the electrical charge may need to be provided by an external source, as the device may not be able to generate sufficient voltage to rearrange the relevant section of the graphene structure and release the (third or) fourth medicaments, or others. Such external sources can be provided in the form of, for example, a portable battery pack, or mains power in a suitable environment, or in a medical facility.

The device of the invention may be used for the treatment of any medical condition where a medicament may be administered topically for absorption through the skin; or, alternatively, for delivery of any active ingredient which can be administered topically. Such medical conditions may include, but are not limited to, the localised and specialised treatment of skin trauma, such as burns, ulcers and skin conditions. The device of the invention is typically in the form of an easy fitting, loose wrap around structure, and may be in the form of a bandage, or in any form which enables it to be wrapped around a body part, such as an arm, leg, torso, abdomen, or head.

The device of the invention also allows for electrical sensor monitoring, larger area dose administration, monitoring and localised medication monitoring.

The device of the invention may itself act as, or contain, a sensor which can provide information on a wound located underneath the device, without the need to remove the device to visually examine the wound. This way, it would be possible for the sensor to inform the patient or medical practitioner that, for example, a burn wound was still dry, and required a further dose of a specific medication; or, alternatively, that a wound has almost healed and in fact did not require any further medication. In contrast with using conventional bandages, this avoids the need to remove the bandage, examine the wound, and then re-apply a fresh bandage to the wound, saving both time, effort and costs. Using the available charge as a power supply, the sensors may be embedded into the structure at regular intervals, and monitoring will be in the form of a change of state indicator where it can be pinpointed by software link up.

The device of the invention may also permit specific affected areas of the human or animal body located under the device to be treated with the one or more medicaments. The device may also activate medication under the skin in the form of microchip electronics or phased release of medication.

Using the sensor information to provide a 'map' the structure can see where the medication is required, further localised medication can be administered by localised increased electronic current. This can be achieved by the placement of bespoke shaped 'patches' over specific areas of the device to allow enhanced localized treatment through thermal, light or electrical stimulation to the device layers underneath. The development of an additional hand held device which would be able to map the structure and provide further electronic charge would enable a controlled increase dose.

Also envisaged is that the device can be used to provide a map of the wound area under the device, without removing the device. This information may be transmitted for example via wireless and bluetooth technology to an information storage device, such as a computer, tablet or an app.

It would also be possible to overlay a further patch, containing a medicament, onto the device of the invention, so the active ingredient therein could also be transported into the patient.

According to one embodiment of the invention, the first layer may comprise from about 5 wt% to about 40 wt% of graphene or graphite, typically from about 10 wt% to about 35 wt%, or from about 15 wt% to about 30 wt%, or from about 20 wt% to about 25 wt%.

According to another embodiment of the invention, the second layer may comprise from about 40 wt% to about 80 wt% of graphene or graphite, typically from about 45 wt% to about 75 wt%, or from about 50 wt% to about 70 wt%, or from about 55 wt% to about 65 wt%.

Also present in the first layer and/or the second layer may be from about 75 to about 25 wt%, or from about 70 to about 30 wt%, or from about 65 to about 35 wt%, or from about 60 to about 40 wt%, or from about 55 to about 45 wt%, of one or more materials possessing medically advanced antibacterial and/or anti-inflammatory properties. Such materials may include, by way of non-limiting example, honey and/or other naturally occurring plant materials.

Advanced wound care is now using nano technology to enable very localised treatment, as a delivery mechanism we would look to adapt and include the most advanced medical solutions and materials such as delivery of collagen, stem cell based products or even graphene based solutions, that can be 'guided' into target areas through very fine needles.

The medical dressing material of the third layer may comprise one or more of a number of different materials, such as but not limited to, a gauze material, a film, gels, a foam, a hydrocolloid, an alginate, a hydrogel, a polysaccharide paste, granules and/or beads, or collagen. Stem cells directly combined with graphene oxide may also provide an example of such advanced medication. Such materials that may be suitable for this layer would be apparent to the skilled medical practitioner and nanomaterial scientist.

Also provided in accordance with the present invention is a fastening apparatus, comprising a sliding tab and a plurality of interlockable teeth, wherein the apparatus is capable of generating an electrical charge by movement of the sliding tab relative to the teeth. The fastening apparatus is typically a zip (or zipper) or the same mechanism can be fastened by overlaying and joining the anode and cathode layers providing a power stimulus. In the zip fastening apparatus, the interlocking teeth are typically alternating anodes and cathodes.

The anodes and cathodes each typically comprise an enhanced level of graphene, working with more recognized materials such as lithium or other developing materials such as zinc oxide or lithium sulphur. The teeth are capable of acting as supercapacitors for rapid charging and release of electrical charge. When the sliding tab is moved in order to bring the teeth into contact with each other, a circuit is closed, photons are excited across the circuit, and an electrical charge is created. The faster the sliding tab is moved, the greater the photons are excited, and the larger the charge is created. This charge can then potentially be dissipated to serve a particular purpose, such as to aid in the administration of one or more medicaments in the device of the invention, as defined herein above.

The material also has adaptive markets in extreme environment clothing such as marine, travel accessories and childcare products, transport and automotive industries where there is a direct requirement for reaction to the wearers environment, *e.g.* hot or cold adjusting capability.

The sliding tab typically comprises, or is made in composite of graphene, and also typically comprises a display enabling a user to see how much charge has been created.

The present invention also provides a method of generating and/or distributing power comprising employing a fastening apparatus as defined hereinabove.

According to another embodiment of the invention, there is provided a wound dressing comprising a device as defined hereinabove.

According to another embodiment of the invention, there is provided a garment comprising a device as defined hereinabove.

According to another embodiment of the invention, there is provided a use of one or more devices as defined hereinabove in the delivery of one or more active ingredients which are suitable for topical administration. Also provided is the use of one or more devices as defined hereinabove in the treatment of skin trauma.

The invention will now be described further by way of example with reference to the following figures which are intended to be illustrative only and in no way limiting upon the scope of the invention.
Figure 1 depicts a battery mechanism showing anode and cathode power interaction used in the invention.
Figure 2 depicts a pictorial view of the zip fastening mechanism used to secure the device of the invention on a person.
Figure 3 depicts a pictorial view of a particular arrangement for the release of a medication from the device of the invention.

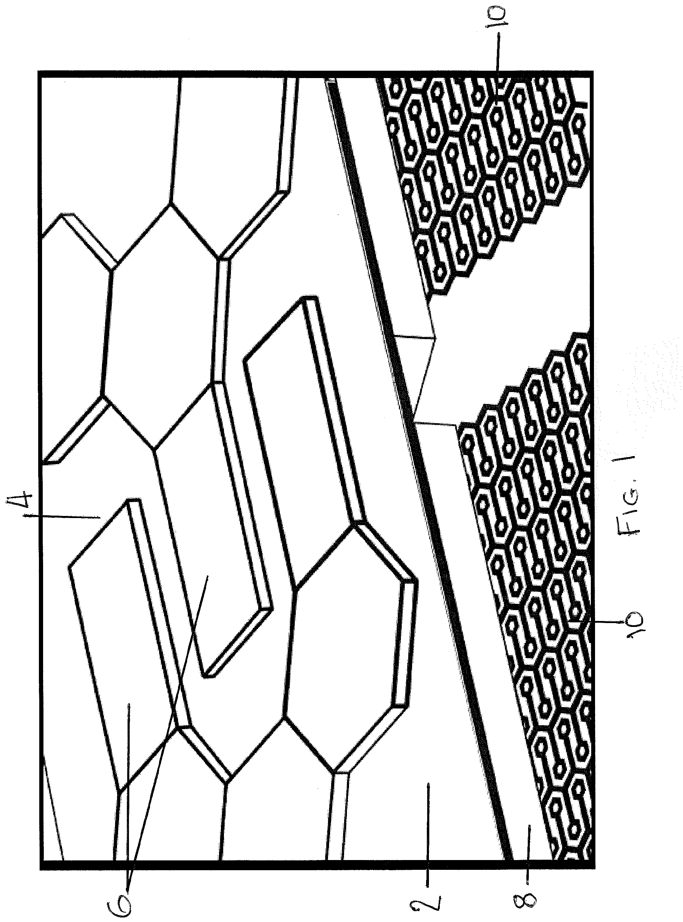

In Figure 1, a close-up view of the first layer 2 with the zip fastening apparatus 4 is shown in its closed configuration, with the interlocking teeth 6. The interlocking teeth 6 constitute alternating anodes and cathodes along the entire length of the zip fastening apparatus 4.

Underneath the first layer 2 and the zip fastening apparatus 4 is the medical dressing layer 8, and the second layer 10, which acts as the delivery layer for the delivery of the medicament or active ingredient.

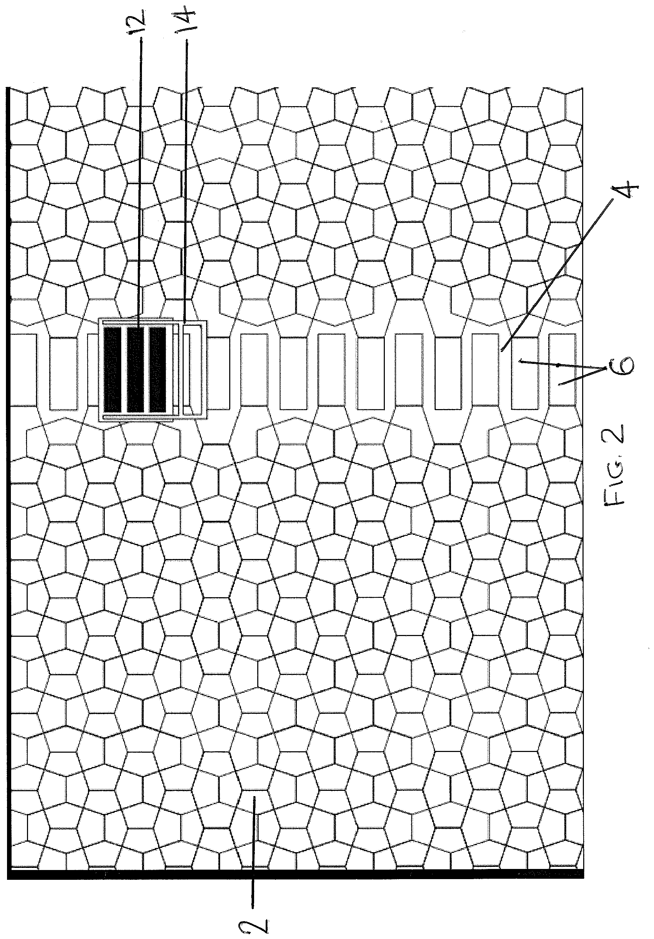

Figure 2 shows a representation of the first layer 2 with the zip fastening apparatus 4, with the zip fastening apparatus 4 in its closed configuration, The interlocking teeth 6 have been brought into contact with each other, thus charging the device. A display screen 12 is visible on the sliding tab 14 of the zip fastening apparatus 4, providing an indication of how much charge has been created and is free to be transferred into the graphene structure to facilitate the release of the medicament or active ingredient.

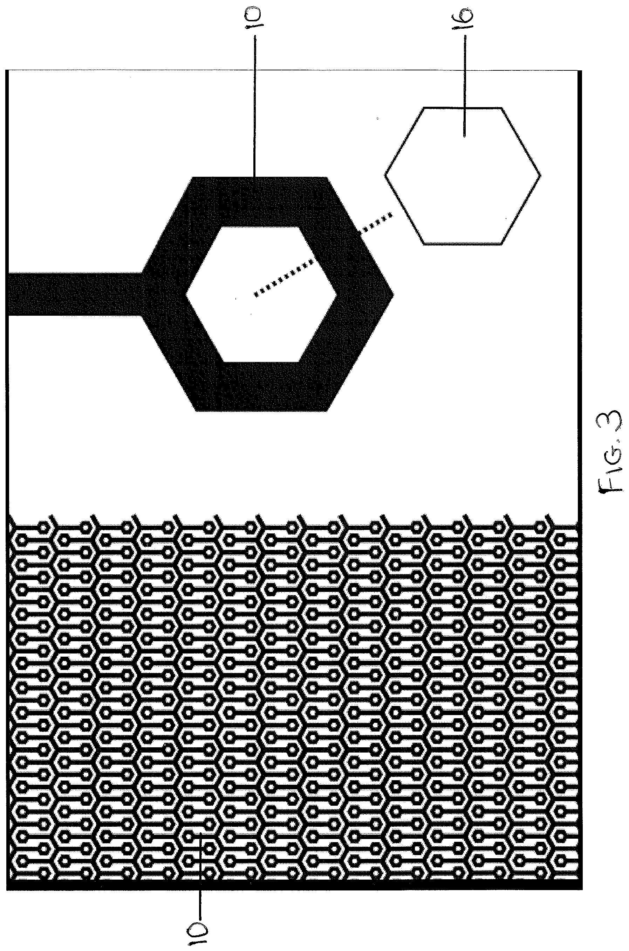

In the left-hand side of Figure 3 can be seen a depiction of the structure of the second layer 10 containing a medicament or active ingredient 16 trapped within the hexagonally-shaped graphene structure. On the right-hand side of Figure 3, an enlarged view of the structure of the second layer 10 is shown. Upon application of charge to the structure of the second layer 10, the structure deforms, and deforms sufficiently to be able to release the medicament or active ingredient 16 so it is then free to enter a person's body and provide the desired medical effect.

It is of course to be understood that the present invention is not intended to be restricted to the foregoing examples which are described by way of example only.

## Claims

1. A device for delivering one or more medicaments or active ingredients to a human or animal body, the device comprising:
i) a first layer (2) comprising graphene and a metal composite material;
ii) a second layer (10) comprising graphene and/or a graphene composite material;
iii) a third layer (8) located between the first layer and second layer, the third layer comprising a medical dressing material; and
iv) one or more medicaments or active ingredients which are releasably contained within the second layer (10);
wherein the one or more medicaments or active ingredients (16) are released from the device upon application of an electric charge to the first and/or second layers by use of a fastening apparatus (4), the fastening apparatus comprising a sliding tab and a plurality of interlockable teeth (6), wherein the apparatus is capable of generating an electrical charge by movement or connection of the sliding tab (14) relative to the teeth (6); or through application to the device of thermal change, body heat, or light to the device.

2. A device according to claim 1, wherein when there is more than one medicament or active ingredients (16), they are released sequentially from the device upon application to the first (2) and/or second layer (10) of electric charges of increasing strength.

3. A device according to claim 1 or claim 2, wherein the graphene in the first layer (2) and/or second layer (10) is structurally modified by the application of an electric charge to the first (2) and/or second layers (10), enabling release of the one or more medicaments or active ingredients.

4. A device according to any preceding claim, wherein the device is in a form which enables it to be wrapped securely around a body part;
optionally wherein the device is fastened around a body part by a zip or overlaying fastener, optionally wherein the zip (4) or overlaying fastener acts as a power source for the device.

5. A device according to claim 4, wherein the zip fastener (4) comprises a sliding tab (14) comprising graphene, and teeth comprising graphene-containing batteries, optionally wherein movement of the sliding tab (14) to bring the teeth (6) into connection with each other creates an electrical charge; and/or
optionally wherein the sliding tab comprises a display enabling a user to see how much charge has been created.

6. A device according to any preceding claim, wherein the application of an electric charge to the first (2) and/or second layer (10) results in an increase in pressure from the device on a body part to which the device is applied.

7. A device according to any preceding claim, wherein the first (2) and/or second layer (10) each further comprise of highly conductive and clinically approved metal composites.

8. A device according to any preceding claim, wherein the first layer (2) comprises from 5 wt% to 40 wt% of graphene or graphite; and/or wherein the second layer (10) comprises from 40 wt% to 80 wt% of graphene or graphite.

9. A device according to any preceding claim, wherein the metal composite material comprises one or more metals selected from copper, zinc, platinum, silver, gold, nickel, gallium, or cobalt, or combinations of any two or more thereof, optionally wherein the metal composite material comprises at least about 40% of one metal by weight of the first layer; optionally wherein the metal is copper; and/or wherein the graphene composite material comprises graphene oxide.

10. A device according to any preceding claim, wherein the third layer (8) comprises one or more materials selected from a gauze material, a film, gels, a foam, a hydrocolloid, an alginate, a hydrogel, a polysaccharide paste, granules and/or beads, or collagen; and/or optionally wherein the device comprises up to about six separate medicaments located in the second layer.

11. A device according to any preceding claim, further comprising a sensor which can provide information on a wound located on a body part underneath the device.

12. A wound dressing or garment comprising one or more devices according to any preceding claim.

13. A device according to any of claims 1-12 suitable for use in the delivery of one or more active ingredients which are suitable for topical administration; or for use in the treatment of skin trauma.

14. A device according to any preceding claim,
wherein the sliding tab (14) of the fastening apparatus (4) comprises graphene, and the teeth each optionally comprise graphene-containing batteries;
optionally wherein movement of the sliding tab (14) to bring the teeth (14) into connection with each other creates an electrical charge;
optionally wherein the sliding tab (14) comprises a display enabling a user to see how much charge has been created.

## Patentansprüche

1. Vorrichtung zur Abgabe eines oder mehrerer Medikamente oder Wirkstoffe an einen menschlichen oder tierischen Körper, wobei die Vorrichtung aufweist:
i) eine erste Lage (2) aufweisend Graphen und Metallverbundmaterial;
ii) eine zweite Lage (10) aufweisend Graphen und/oder ein Graphenverbundmaterial;
iii) eine dritte Lage (8), die zwischen der ersten Lage und der zweiten Lage angeordnet ist, wobei die dritte Lage ein medizinisches Verbandmaterial aufweist; und
iv) ein oder mehrere Arzneimittel oder Wirkstoffe, welche freisetzbar in der zweiten Lage (10) enthalten sind;
wobei das eine oder mehrere Arzneimittel oder Wirkstoffe (16) von der Vorrichtung durch Anlegen einer elektrischen Ladung an die erste und/oder zweite Lage freigesetzt werden durch Verwendung einer Befestigungseinrichtung (4), wobei die Befestigungseinrichtung eine Schieblasche und eine Vielzahl von ineinandergreifenden Zähnen (6) aufweist, wobei die Einrichtung in der Lage ist, eine elektrische Ladung durch Bewegen oder Verbinden der Schieblasche (14) relativ zu den Zähnen (6) zu erzeugen; oder durch Aufbringen an die Vorrichtung von Wärmeenergie, Körperwärme oder Licht an die Vorrichtung.

2. Vorrichtung nach Anspruch 1,
wobei wenn es mehr als ein Medikament oder Wirkstoff (16) gibt, werden sie sequenziell von der Vorrichtung freigesetzt durch Anwenden an die ersten (2) und/oder zweiten Lage (10) von elektrischer Ladung mit ansteigender Stärke.

3. Vorrichtung nach Anspruch 1 oder 2,
wobei das Graphen in der ersten Lage (2) und/oder der zweiten Lage (10) durch Anwendung einer elektrischen Ladung auf die erste (2) und/oder zweite Schicht (10) modifiziert wird, wodurch das Freisetzen des ein oder mehreren Medikamentes oder Wirkstoffes möglich wird.

4. Vorrichtung nach einem der vorangehenden Ansprüche,
wobei die Vorrichtung in einer Form ist, welche es ermöglicht, dass sie sicher um ein Körperteil gewickelt wird;
optional wenn die Verrichtung um ein Körperteil mit einem Reißverschluss oder einem darüber liegenden Verschluss befestigt ist, optional wenn der Reißverschluss (4) oder der darüber liegende Verschluss als Energiequelle für die Vorrichtung dient.

5. Vorrichtung nach Anspruch 4,
wobei der Reißverschluss (4) eine Schieblasche (14) mit Graphen und Zähne mit Graphen beinhaltenden Batterien aufweist optional, wobei Bewegung der Schieblasche (14) an den Zähnen (6) in Verbindung miteinander eine elektrische Ladung erzeugt und/oder
optional wobei die Schieblasche eine Anzeige aufweist, welche es dem Verwender ermöglicht zu sehen, wieviel Ladung erzeugt wurde.

6. Vorrichtung nach einem der vorangehenden Ansprüche,
wobei die Anwendung einer elektrischen Ladung an die erste (2) und/oder zweite Lage (10) in einer Erhöhung des Druckes von der Vorrichtung auf einen Körperteil, an dem die Vorrichtung angebracht ist, resultiert.

7. Vorrichtung nach einem der vorangehenden Ansprüche,
wobei die erste Lage (2) und/oder zweite Lage (10) jeweils weiterhin aus hoch leitfähigen und klinisch zugelassenen Metallverbundwerkstoffen besteht.

8. Vorrichtung nach einem der vorangehenden Ansprüche,
wobei die erste Lage (2) von etwa 5 Gew.% bis 40 Gew.% Graphen oder Graphit aufweist; und/oder wobei die zweite Lage (10) von 40 Gew.% bis 80 Gew.% Graphen oder Graphit aufweist.

9. Vorrichtung nach einem der vorangehenden Ansprüche,
wobei das Metallverbundmaterial aufweist eine oder mehrere Metalle ausgewählt aus Kupfer, Zink, Platin, Silber, Gold, Nickel, Gallium oder Kobalt oder eine Kombination von zwei oder mehreren davon aufweist, optional wobei der Metallverbundstoff aufweist mindestens etwa 40% eines Metalls bezogen auf das Gewicht der ersten Lage; optional wobei das Metall Kupfer ist; und/oder wobei das Graphenverbundmaterial Graphen-Oxid aufweist.

10. Vorrichtung nach einem der vorangehenden Ansprüche,
wobei die dritte Lage (8) aufweist ein oder mehr Materialien ausgewählt aus Gaze-Material, einem Film, Gelen, einem Schaum, einem Hydrokolloid, einem Alginat, einem Hydrogel, einer Polysacharid-Paste, Granulat und/oder Perlen oder Kollagen; und/oder optional wobei die Vorrichtung bis zu ungefähr sechs separate Medikamente, welche in der zweiten Lage lokalisiert sind, aufweist.

11. Vorrichtung nach einem der vorangehenden Ansprüche des Weiteren aufweisend einen Sensor, welcher Informationen über eine Wunde liefern kann, welche sich an einem Körperteil unterhalb der Vorrichtung befindet.

12. Wundverband oder Kleidungsstück aufweisend eine oder mehrere Vorrichtungen nach einem der vorangehenden Ansprüche.

13. Vorrichtung nach einem der Ansprüche 1 bis 12,
geeignet zur Verwendung in der Verabreichung von einem oder mehreren Wirkstoffen, welche zur topischen Verabreichung geeignet sind oder zur Verwendung in der Behandlung von Hautverletzungen.

14. Vorrichtung nach einem der vorangehenden Ansprüche,
wobei die Schublasche (14) der Befestigungseinrichtung (4) Graphen aufweist und die Zähne jeweils optional Graphen beinhaltende Batterien aufweisen; optional wobei das Bewegen der Schublasche (14) um die Zähne (14) in Kontakt miteinander zu bringen eine elektrische Ladung erzeugt;
optional wobei die Schublasche (14) eine Anzeige aufweist, welche es dem Benutzer ermöglicht zu sehen, wieviel Ladung erzeugt wurde.

## Revendications

1. Dispositif d'administration d'un médicament ou de plusieurs médicaments ou d'ingrédients actifs à un corps humain ou animal, le dispositif comprenant :
i) une première couche (2), comprenant du graphène et un matériau composite de métal ;
ii) une deuxième couche (10), comprenant du graphène et/ou un matériau composite de graphène ;
iii) une troisième couche (8) placée entre la première couche et la deuxième couche, la troisième couche comprenant une matière de compresse médicale ; et
iv) un ou plusieurs médicaments ou ingrédients actifs, qui sont contenus de manière libérable au sein de la deuxième couche (10) ;
dans lequel le médicament ou les plusieurs médicaments ou les ingrédients (16) actifs sont libérés du dispositif par application d'une charge électrique à la première et/ou à la deuxième couche, en utilisant un système (4) de fermeture, le système (4) de fermeture comprenant une patte coulissante et une pluralité de dents (6) enclenchables entre elles, le système étant apte à produire une charge électrique par mouvement ou connexion de la patte (14) coulissante par rapport aux dents (6) ou par application au dispositif d'un changement thermique, de la chaleur du corps ou de la lumière au dispositif.

2. Dispositif suivant la revendication 1, dans lequel, lorsqu'il y a plus d'un médicament ou d'ingrédients (16) actifs, ils sont libérés séquentiellement du dispositif par application à la première (2) et/ou à la deuxième couche (10) de charge électrique de force croissante.

3. Dispositif suivant la revendication ou la revendication 2, dans lequel le graphène de la première couche (2) et/ou de la deuxième couche (10) est modifié structuralement par l'application d'une charge électrique à la première (2) et/ou à la deuxième couche (10), en permettant une libération du un médicament ou des plusieurs médicaments ou ingrédients actifs.

4. Dispositif suivant l'une quelconque des revendications précédentes, dans lequel le dispositif est sous la forme, qui permet d'en envelopper, d'une manière sûre, une partie du corps ;
dans lequel, éventuellement, le dispositif est fixé autour d'une partie du corps par une fermeture à glissière ou par un dispositif de fermeture, qui le surmonte, dans lequel, le cas échéant, la fermeture (4) à glissière ou le dispositif de fixation, qui le surmonte, sert de source de courant au dispositif.

5. Dispositif suivant la revendication 4, dans lequel le dispositif (4) de fermeture à glissière comprend une patte (4) coulissante comprenant du graphène et des dents comprenant des batteries contenant du graphène, dans lequel, éventuellement, un déplacement de la patte (14) coulissante, pour mettre les dents (6) en liaison les unes avec les autres, crée une charge électrique et/ou dans lequel, éventuellement, la patte coulissante comprend un affichage permettant à un utilisateur de voir combien de charge a été créée.

6. Dispositif suivant l'une quelconque des revendications précédentes, dans lequel l'application d'une charge électrique à la première (2) et/ou à la deuxième couches (10) se traduit par une augmentation de la pression du dispositif sur une partie du corps à laquelle le dispositif est appliqué.

7. Dispositif suivant l'une quelconque des revendications précédentes, dans lequel la première (2) et/ou la deuxième couches (10) comprennent chacune, en outre, des composites de métal très conducteurs et approuvés cliniquement.

8. Dispositif suivant l'une quelconque des revendications précédentes, dans lequel la première couche (2) comprend de 5% en poids à 40% en poids de graphène ou de graphite et/ou dans lequel la deuxième couche (10) comprend de 40% en poids à 80% en poids de graphène ou de graphite.

9. Dispositif suivant l'une quelconque des revendications précédentes, dans lequel le matériau composite de métal comprend un ou plusieurs métaux choisis parmi le cuivre, le zinc, le platine, l'argent, l'or, le nickel, le gallium ou le cobalt, ou des combinaison de deux ou plusieurs d'entre eux, dans lequel, éventuellement, le matériau composite de métal comprend au moins environ 40% d'un métal en poids de la première couche, dans lequel, éventuellement, le métal est du cuivre, et/ou dans lequel le matériau composite de graphène comprend de l'oxyde de graphène.

10. Dispositif suivant l'une quelconque des revendications précédentes, dans lequel la troisième couche (8) comprend un ou plusieurs matériaux choisis parmi un matériau de gaze, un film, des gels, une mousse, un hydrocolloïde, un alginate, un hydrogel, une pâte de polysaccharide, des granules et/ou des perles, ou du collagène, et/ou dans lequel, éventuellement, le dispositif comprend jusqu'à environ six médicaments distincts placés dans la deuxième couche.

11. Dispositif suivant l'une quelconque des revendications précédentes, comprenant, en outre, un capteur, qui peut fournir de l'information sur une blessure sur une partie du corps en dessous du dispositif.

12. Pansement de blessure ou vêtement comprenant un ou plusieurs dispositifs suivant l'une quelconque des revendications précédentes.

13. Dispositif suivant l'une quelconque des revendications 1 à 12, propre à être utilisé dans l'administration d'un ou plusieurs ingrédients actifs, qui conviennent pour une administration topique, ou à être utilisé dans le traitement de plaies de la peau.

14. Dispositif suivant l'une quelconque des revendications précédentes,
dans lequel la patte (14) coulissante du système (4) de fermeture comprend du graphène, et chaque dent comprend, éventuellement, des batteries contenant du graphène ;
dans lequel, éventuellement le déplacement de la patte (14) coulissante, pour mettre les dents (14) en liaison les unes avec les autres, crée une charge électrique,
dans lequel, éventuellement, la patte (14) coulissante comprend un affichage permettant à un utilisateur de voir combien de charge a été créée.
